# EUROPÄISCHE PATENTSCHRIFT

(11) **EP 2 736 562 B1**
(45) Veröffentlichungstag und Bekanntmachung des Hinweises auf die Patenterteilung: **08.06.2016**
(21) Anmeldenummer: 12745626.7
(22) Anmeldetag: 27.07.2012
(51) Int. Cl.: A61M 1/36

(54) **BERÜHRSCHUTZVORRICHTUNG FÜR MEDIZINISCHE FLUIDFÜHRENDE KASSETTE UND KASSETTE**
CONTACT PROTECTION DEVICE FOR MEDICAL FLUID-CARRYING CARTRIDGE AND CARTRIDGE
DISPOSITIF DE PROTECTION CONTRE LE CONTACT POUR CASSETTE CONDUISANT DES FLUIDES MÉDICAUX, AINSI QUE CASSETTE

(30) Priorität: 29.07.2011 DE 102011108781; 29.07.2011 US 201161512952 P
(43) Veröffentlichungstag der Anmeldung: 04.06.2014
(73) Patentinhaber: Fresenius Medical Care Deutschland GmbH, 61352 Bad Homburg (DE)
(72) Erfinder: GERLACH, Daniel, 60316 Frankfurt am Main (DE); HAECKER, Juergen, 61267 Neu-Anspach (DE); KREBER, Stefan, 66113 Saarbrücken (DE); LEICK, Lothar, 66663 Merzig-Ballern (DE); SCHULZ, Wolfgang, 66606 St. Wendel (DE)
(74) Vertreter: Bobbert, Cornelius
(86) Internationale Anmeldenummer: PCT/EP2012/003189
(87) Internationale Veröffentlichungsnummer: WO 2013/017235

(56) Entgegenhaltungen:
- US-A- 5 441 636
- US-A1- 2008 093 246
- US-A1- 2010 274 168
- US-A1- 2011 064 608
- US-A1- 2011 098 635

## Beschreibung

Die vorliegende Erfindung betrifft eine medizinische fluidführende Blutkassette (kurz: Kassette) gemäß Anspruch 1 mit einer Berührschutzvorrichtung zum Abdecken einer Konnektionsstelle der Blutkassette für eine medizinische Fluidbehandlung, beispielsweise zum Abdecken einer Substituatstelle der Blutkassette wie sie in Dialysegeräten verwendet werden kann.

Eine medizinische, fluidführende Blutkassette wird z.B. in US 2008/093246 (A1) beschrieben.

Bei der Handhabung von fluidführenden Kassetten, beispielsweise von Blutkassetten, durch das medizinische Personal besteht stets die Gefahr, dass beim Auspacken einer Kassette aus der Sterilverpackung versehentlich Konnektionsstellen, welche während der Behandlung des Patienten zur Zugabe eines Fluids in die Kassette (kurz: Fluidzugabestellen der Kassette) genutzt wird, mit dem Finger oder einem Gegenstand berührt und somit kontaminiert werden. Aus der Praxis sind bereits verschiedene Lösungen hierfür bekannt, zum Beispiel in Form von lösbar verklebten Schutzfolien.

Eine Aufgabe der vorliegenden Erfindung ist es, eine weitere medizinische fluidführende Blutkassette mit Berührschutzvorrichtung zum Abdecken einer Konnektionsstelle der Blutkassette für eine medizinische Fluidbehandlung vorzuschlagen.

Die erfindungsgemäße Aufgabe wird durch eine medizinische fluidführende Blutkassette mit einer Berührschutzvorrichtung mit den Merkmalen des Anspruchs 1 gelöst.

Die Erfindung betrifft eine medizinische fluidführende Blutkassette mit wenigstens einer Berührschutzvorrichtung gemäß Anspruch 1.

Die Berührschutzvorrichtung zum Abdecken einer Konnektionsstelle einer medizinischen fluidführenden Kassette für eine medizinische Fluidbehandlung weist wenigstens eine Abdeckeinrichtung zum Abdecken der Konnektionsstelle vor Gebrauch der Kassette, d. h. bevor die Kassette an einer Fluidbehandlungsvorrichtung angebracht wird, auf. Sie weist ferner wenigstens einen Verbindungsabschnitt zum lösbaren Verbinden oder Halten der Berührschutzvorrichtung an der Kassette auf.

Die wenigstens eine Abdeckeinrichtung der Berührschutzvorrichtung ist über wenigstens eine Konnektionsstelle einer fluidführenden Kassette platzierbar und breit genug, um diese Konnektionsstelle, oder mehrere Konnektionsstellen, der Kassette derart abzudecken, dass bei aufgesetzter Berührschutzvorrichtung bei üblichem Gebrauch kein Abschnitt der Konnektionsstelle durch Berührung mit den Fingern kontaminiert werden kann. Die Abdeckeinrichtung kann alle dem Stand der Technik bekannten Formen haben, beispielsweise kann sie plattenförmig sein. Sie kann rechteckig, halbrund oder dergleichen ausgestaltet sein, usw. Die Größe der Abdeckeinrichtung kann in Abhängigkeit zur Größe der im Einzelfall zu schützenden Konnektionsstelle ausgewählt sein.

Vorteilhafte Weiterentwicklungen der vorliegenden Erfindung sind jeweils Gegenstand von Unteransprüchen und Ausführungsformen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen können eines oder mehrere der im Folgenden genannten Merkmale aufweisen.

Die Abdeckeinrichtung wird in manchen erfindungsgemäßen Ausführungsformen mittels - insbesondere ausschließlich - Spannung oder Materialsteifigkeit an der Kassette gehalten. Zwischen der Abdeckeinrichtung und der Kassette besteht in diesen Ausführungsformen keine stoffschlüssige Verbindung.

In bestimmten erfindungsgemäßen Ausführungsformen ist die Berührschutzvorrichtung ausgestaltet und/oder verbunden, um zerstörungsfrei von der Kassette abnehmbar zu sein. Die Berührschutzvorrichtung wird in einigen erfindungsgemäßen Ausführungsformen mittels eines Verbindungsabschnitts an der Kassette gehalten. Der Verbindungsabschnitt sorgt für eine lösbare und stabile Verbindung der Berührschutzvorrichtung an der Kassette. Diese Verbindung kann klebstofffrei erfolgen. Sie kann ein Einrasten, Arretieren, Einbringen, Einklemmen, Einschrauben, ein Halten mittels physikalischer Kräfte oder eine sonstige aus dem Stand der Technik bekannte Art des lösbaren Verbindens sein.

Die Berührschutzvorrichtung ist in manchen erfindungsgemäßen Ausführungsformen ausgestaltet, um beliebig oft mit der Kassette verbindbar und wieder lösbar zu sein.

In gewissen Ausführungsformen ist die erfindungsgemäße Berührschutzvorrichtung plattenförmig. In manchen erfindungsgemäßen Ausführungsformen ist die Berührschutzvorrichtung einteilig oder einstückig. Es ist aber für den Fachmann erkennbar, dass die Berührschutzvorrichtung sowohl jede beliebige aus dem Stand der Technik bekannte Form haben kann, um ihre Funktion zu erfüllen, als auch ein- oder mehrstückig oder -teilig gefertigt werden kann.

In manchen erfindungsgemäßen Ausführungsformen wird die Berührschutzvorrichtung allein durch den Verbindungsabschnitt an der Kassette gehalten.

In manchen erfindungsgemäßen Ausführungsformen weist die Berührschutzvorrichtung wenigstens eine Kodierstruktur zum formbedingt kodierten Anordnen der Berührschutzvorrichtung an der fluidführenden Kassette auf. Das kodierte Anordnen kann zu einer stabilen Verbindung der Berührschutzvorrichtung an der Kassette beitragen. Hierzu hat die Kodierstruktur eine Form, die beispielsweise mit der Form oder Kontur eines Randes (oder eines anderen Abschnitts) der Kassette korreliert oder sich mit diesem ergänzt oder komplementär zu diesem ist. Das bewirkt einerseits, dass die Verbindung zwischen der Berührschutzvorrichtung und der im Randbereich ebenfalls in der Form der Kodierstruktur verlaufenden Kassette stabil ist. So kann die Kodierstruktur beispielsweise das Verrutschen der Berührschutzvorrichtung oder deren Verdrehen um die Achse ihrer Verbindung mit der Kassette, beispielsweise um den Verbindungsabschnitt, verhindern. Andererseits kann die Kodierstruktur vorteilhaft verhindern, dass die Berührschutzvorrichtung während des Herstellungsprozesses in einer anderen Position zur Kassette als der beabsichtigten an der Kassette angebracht wird. Somit kann ein für den beabsichtigten Berührschutz unwirksames Anbringen der Berührschutzvorrichtung vorteilhaft verhindert werden.

In einigen erfindungsgemäßen Ausführungsformen ist die Kodierstruktur der Berührschutzvorrichtung wellenförmig oder weist einen wellenförmigen Abschnitt auf. Es ist für den Fachmann erkennbar, dass die Kodierstruktur jede erdenkliche Form aufweisen kann, mit welcher sie wenigstens eine der o. g. Funktionen oder Vorteile erfüllen kann. Beispielsweise kann sie eine gerade Linie, ein Zickzackmuster oder sonstiges gestuftes Muster aufweisen. Sie kann ein kontinuierliches oder ein nicht-kontinuierliches Muster aufweisen, usw.

In gewissen erfindungsgemäßen Ausführungsformen ist die Kodierstruktur der Berührschutzvorrichtung eine zu diesem Zweck vorgesehene Aussparung in wenigstens einem Abschnitt oder auf einer Seite der Berührschutzvorrichtung.

In manchen erfindungsgemäßen Ausführungsformen weist die Berührschutzvorrichtung wenigstens eine Fixierungseinrichtung zum lösbaren Aufnehmen, Begrenzen oder Fixieren von Zu- und/oder Ablaufschläuchen der fluidführenden Kassette auf.

Hierzu hält die Fixierungseinrichtung die Schläuche in einigen erfindungsgemäßen Ausführungsformen in einer gewünschten Stellung relativ zur Kassette. Die Fixierungseinrichtung kann so die Beschädigung der Schläuche durch Abknicken oder deren Kontamination durch ungewolltes Lösen der Schläuche von der Kassette verhindern.

In bestimmten erfindungsgemäßen Ausführungsformen ist die Fixierungseinrichtung mehrteilig ausgeführt. Zwischen den einzelnen Abschnitten der mehrteiligen Fixierungseinrichtung können weitere Gegenstände wie beispielsweise Schlauchklemmen, welche wiederum mit den vorgenannten Schläuchen verbunden sein können, vorübergehend mittels Reibhaftung oder Rastwirkung gehalten werden. Dies kann einem Schutz der Gegenstände wenigstens bis zum Gebrauch der Kassette dienen.

In einigen erfindungsgemäßen Ausführungsformen des Berührschutzes ist die Fixierungseinrichtung auf nur einer Seite der Berührschutzvorrichtung angeordnet. Dabei erstreckt sie sich nur auf eine Seite.

In manchen erfindungsgemäßen Ausführungsformen weist die Berührschutzvorrichtung eine vollständig ebene oder ausschließlich ebene Fläche auf. Diese Fläche ist im Gebrauch der Berührschutzvorrichtung der Konnektionsstelle abgewandt. Die ebene Fläche verhindert vor dem Gebrauch der Kassette vorteilhaft ein ungewolltes Verhaken der Berührschutzvorrichtung mit anderen Gegenständen oder ihr Hängenbleiben an diesen, wodurch die Berührschutzvorrichtung unbeabsichtigt von der Kassette gelöst werden könnte.

In gewissen erfindungsgemäßen Ausführungsformen weist die Berührschutzvorrichtung wenigstens einen Griffabschnitt zum Greifen der Schutzvorrichtung auf, um die Berührschutzvorrichtung mit der Hand von der fluidführenden Kassette zu lösen. Mittels des Griffabschnitts entfernt oder löst der Benutzer die Berührschutzvorrichtung von der Kassette vor dem Einlegen der Kassette in eine Fluidbehandlungsvorrichtung.

Der Griffabschnitt kann jede dem Stand der Technik bekannte Ausgestaltung aufweisen, insbesondere eine solche, welche ein rutschsicheres Halten oder Greifen des Griffabschnittes erleichtert oder ermöglicht. Beispielsweise kann der Griffabschnitt als Ring, Noppen, Lasche usw. ausgestaltet sein oder eine Riffelung oder raue Oberfläche aufweisen.

In manchen erfindungsgemäßen Ausführungsformen der Berührschutzvorrichtung liegt die Abdeckeinrichtung nicht auf dem Griffabschnitt vor, sondern auf einem anderen Abschnitt der Berührschutzvorrichtung. Das bewirkt, dass die Abdeckeinrichtung nicht angefasst werden muss, um die Berührschutzvorrichtung von der Kassette zu lösen. Es minimiert somit die Gefahr der versehentlichen Kontamination während des Lösens der Berührschutzvorrichtung.

In bestimmten Ausführungsformen der erfindungsgemäßen Berührschutzvorrichtung liegt der Griffabschnitt auf einem der Abdeckeinrichtung entgegengesetzten Abschnitt der Berührschutzvorrichtung vor. Dies kann zu einem kontaminationslosen Lösen der Berührschutzvorrichtung von der Kassette beitragen.

In manchen erfindungsgemäßen Ausführungsformen der medizinischen fluidführenden Kassette ist die Berührschutzvorrichtung klebstofffrei mit der Kassette verbunden.

In einigen erfindungsgemäßen Ausführungsformen weist die Berührschutzvorrichtung einen Abschnitt auf, welcher über einen Rand der Kassette hinausragt.

In manchen erfindungsgemäßen Ausführungsformen ist der Griffabschnitt der Berührschutzvorrichtung auf dem über den Rand der Kassette hinausragenden Abschnitt angeordnet, um dem Benutzer das Entfernen der Berührschutzvorrichtung von der Kassette zu erleichtern.

In gewissen erfindungsgemäßen Ausführungsformen der Berührschutzvorrichtung ist deren Verbindungsabschnitt in wenigstens eine Halteöffnung oder eine Zentrieröffnung der Kassette eingebracht, um die Berührschutzvorrichtung - ggf. ausschließlich hierdurch - mit der Kassette zu verbinden. Diese Halteöffnung oder Zentrieröffnung der Kassette kann vorgesehen sein, um mit einer Zentrier- oder Halteeinrichtung einer Fluidbehandlungsvorrichtung verbunden zu werden. Sie dient beispielsweise dem Halten oder Zentrieren der Kassette an der Fluidbehandlungsvorrichtung im Gebrauch, beispielsweise während einer Dialysebehandlung.

In manchen erfindungsgemäßen Ausführungsformen verhindert der Verbindungsabschnitt der Berührschutzvorrichtung im Gebrauch das Anbringen der Kassette an einer Fluidbehandlungsvorrichtung oder das Einlegen der Kassette hierin. Dies wird beispielsweise dadurch erreicht, dass, wenn die Berührschutzvorrichtung mit der Kassette verbunden ist, der Verbindungsabschnitt in der Zentrieröffnung oder Halteöffnung der Kassette steckt. Da die Kassette mit aufgesetzter Berührschutzvorrichtung nicht an der Fluidbehandlungsvorrichtung angebracht werden kann, weil ihre Zentrieröffnung nicht frei liegt, muss die Berührschutzvorrichtung vorher bewusst entfernt werden.

In weiteren erfindungsgemäßen Ausführungsformen ist die Berührschutzvorrichtung derart ausgestaltet, beispielsweise durch ihre Dimensionen oder ihre Art der Verbindung mit der Kassette, dass die Kassette mit aufgesetzter Berührschutzvorrichtung zwar an der Fluidbehandlungsvorrichtung angebracht werden kann, jedoch etwa eine Abdeckung, oder beispielsweise eine Maschinentür der Vorrichtung, nicht geschlossen werden kann, ohne dass zuvor die Berührschutzvorrichtung von der Kassette gelöst wird. Auch dies dient dazu sicherzustellen, dass die Berührschutzvorrichtung vor Gebrauch der Kassette zuverlässig entfernt wird.

In einigen vorteilhaften erfindungsgemäßen Ausführungsformen ist die Berührschutzvorrichtung zusammen mit der Kassette sterilisiert. Das Sterilisationsverfahren der Kassette findet in diesen Ausführungsformen mit aufgesetzter Berührschutzvorrichtung statt. Dies ist erfindungsgemäß möglich, da in solchen Ausführungsformen zwischen der Abdeckeinrichtung der Berührschutzvorrichtung - in ihrem über der Konnektionsstelle angeordneten Zustand - und der Kassette und/oder der Konnektionsstelle keine stoffschlüssige oder dampfundurchlässige Verbindung besteht. Somit wird die Dampfdurchgängigkeit der Berührschutzvorrichtung sichergestellt. Die Konnektionsstelle kann selbst bei aufgesetzter Berührschutzvorrichtung bzw. Abdeckeinrichtung erfolgreich sterilisiert werden, beispielsweise mit Heißdampf.

Dieser Vorteil kann sich auch ergeben, wenn Berührschutzeinrichtung und Kassette aus demselben Material oder aus ähnlichen Materialien sind, jedenfalls aus Materialien, welche mit demselben Sterilisationsverfahren, mit welchem auch die Kassette sterilisierbar ist, sterilisiert werden können.

In manchen erfindungsgemäßen Ausführungsformen ist die wenigstens eine Konnektionsstelle, welche mittels der Abdeckeinrichtung abgedeckt ist oder wird, eine Verbindungsstelle für eine Substituatleitung.

In einer weiteren erfindungsgemäßen Ausführungsform ist die medizinische fluidführende Kassette als Blutkassette, beispielsweise für die Dialysebehandlung, insbesondere zur Hämodialyse, zur Hämofiltration, zur Hämodiafiltration, zur Peritonealdialyse, zur Akutdialyse, usw. ausgestaltet.

Manche oder alle erfindungsgemäßen Ausführungsformen können einen, mehrere oder alle der oben und/oder im Folgenden genannten Vorteile aufweisen.

Vorteilhaft ist das Lösen der Berührschutzvorrichtung ohne nennenswerte Kraftanwendung aufgrund der o. g. Ausgestaltung der Berührschutzvorrichtung möglich.

In einigen vorteilhaften Ausführungsformen besteht ein vermindertes Risiko der unbeabsichtigten Kontamination der Konnektionsstelle der Kassette, etwa durch Berühren mit der Hand, da der Griffabschnitt der Berührschutzvorrichtung ausreichend weit von der Abdeckeinrichtung beabstandet ist.

Die Berührschutzvorrichtung ist in vorteilhaften Ausführungsformen aus demselben Material wie die Kassette gefertigt. Somit kann die Berührschutzvorrichtung denselben Sterilisationsverfahren wie die Kassette unterzogen werden. Zudem kann sie auf die gleiche Weise wie die Kassette oder gemeinsam mit dieser entsorgt werden.

Das Lösen der Berührschutzvorrichtung von der Kassette kann in vorteilhaften Ausführungsformen einhändig erfolgen.

In bestimmten erfindungsgemäßen Ausführungsformen weist die Berührschutzvorrichtung entsprechend weiche oder flexible Materialien oder gerundete Kanten derart auf, dass eine Verletzungsgefahr für den Benutzer während ihrer Betätigung, sowie die Gefahr der Beschädigung anderer Bestandteile der Berührschutzvorrichtung oder der Kassette durch beispielsweise scharfe Konturen minimiert sind.

Durch den möglichen Verzicht auf die Verwendung von Klebstoff zum Anbringen der Berührschutzvorrichtung an der Kassette können bei ihrem Lösen von der Kassette keine Klebstoffreste an der Kassette verbleiben und ggf. über die Konnektionsstelle mit in das Kassetteninnere gelangen.

Anders als z. B. bei der Verwendung von Sterilitätskappen besteht erfindungsgemäß keine Gefahr des versehentlichen Berührens der Konnektionsstelle beim Auspacken der Kassette.

Anders als bei Schutzfolien und Sterilitätskappen kann die Berührschutzvorrichtung beim Sterilisieren der Kassette aufgesetzt sein. Ein separates Sterilisieren von Berührschutzvorrichtung und Kassette mit anschließendem Zusammenfügen ist vorteilhaft nicht erforderlich.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:
- Fig. 1: zeigt die Berührschutzvorrichtung von unten, mit Blick auf ihre im Gebrauch zur fluidführenden Kassette hin gewandte Seite;
- Fig. 2: zeigt eine seitliche Darstellung der Berührschutzvorrichtung in einem Schnitt entlang der gestrichelten Linie aus Fig. 1;
- Fig. 3: zeigt eine Draufsicht auf die Berührschutzvorrichtung der Fig. 1, auf ihre im Gebrauch von der fluidführenden Kassette abgewandte Seite;
- Fig. 4: zeigt die Berührschutzvorrichtung der vorangegangenen Figuren in perspektivischer Darstellung mit Blick auf die in Fig. 1 gezeigte Unterseite; und
- Fig. 5: zeigt eine perspektivische Darstellung der Berührschutzvorrichtung der vorangegangenen Figuren im Gebrauch an einer erfindungsgemäßen fluidführenden Blutkassette.

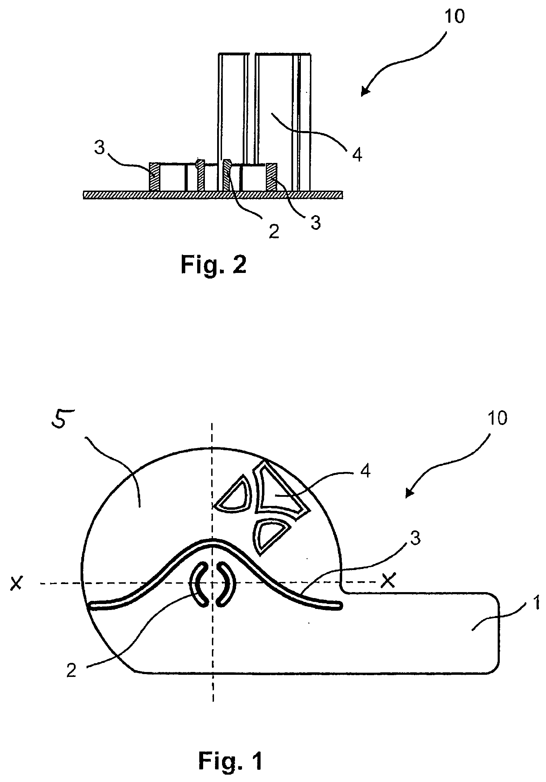

Fig. 1 zeigt eine mögliche Ausgestaltung der Berührschutzvorrichtung 10. Die Berührschutzvorrichtung 10 weist eine Abdeckeinrichtung 1, einen Verbindungsabschnitt 2, eine Kodierstruktur 3, eine Fixierungseinrichtung 4 und einen Griffabschnitt 5 auf.

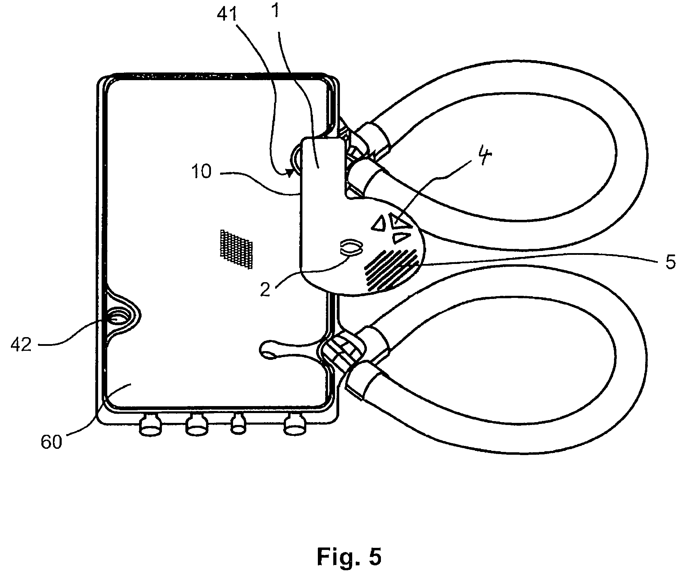

Dabei ist die Berührschutzvorrichtung 10 hier beispielhaft als großflächige Platte oder Scheibe mit einem breiteren Abschnitt, welcher den Verbindungsabschnitt 2 zur Verbindung der Berührschutzvorrichtung 10 trägt, und einem schmaleren Abschnitt, welcher im Gebrauch als Abdeckeinrichtung 1 eine in Fig. 1 nicht gezeigte Konnektionsstelle 41 (siehe Fig. 5) abdeckt, dargestellt. Die Abdeckeinrichtung 1 ist breit genug, um die Konnektionsstelle 41 einer fluidführenden Kassette 60 (in Fig. 1 nicht dargestellt, siehe aber Fig. 5), mittels welcher eine Fluidverbindung zur Kassette hergestellt werden kann, komplett abzudecken. Mittels der Abdeckeinrichtung 1 wird somit ein unbeabsichtigtes Berühren der Konnektionsstelle 41 der Kassette 60 vor deren Gebrauch, d. h. vor dem Anbringen der Kassette 60 an einer Fluidbehandlungsvorrichtung, verhindert. Mittels der Abdeckeinrichtung 1 wird ein unbeabsichtigtes Berühren der Konnektionsstelle 41 auch noch während des Entfernens der Berührschutzvorrichtung 10 verhindert.

Die Abdeckeinrichtung 1 wird mittels Spannung oder über die Materialsteifigkeit der Berührschutzvorrichtung 10 an der Kassette 60 gehalten. Die Berührschutzvorrichtung 10 wird wiederum mittels des Verbindungsabschnitts 2 an der Kassette 60 gehalten.

Der Verbindungsschnitt 2 sorgt für eine lösbare und stabile Verbindung der Berührschutzvorrichtung 10 an der Kassette 60. Diese Verbindung ist klebstofffrei und kann ein Einrasten, Arretieren, Einbringen, Einklemmen, Einschrauben oder eine sonstige aus dem Stand der Technik bekannte Art des lösbaren Verbindens sein. Der Verbindungsabschnitt 2 ist in Fig. 2 eine Einsteck- oder Rastverbindung.

In einigen erfindungsgemäßen Ausführungsformen wird die Berührschutzvorrichtung 10 allein durch den Verbindungsabschnitt 2 an der Kassette 60 gehalten, wie dies auch in den Figuren gezeigt ist. Zwischen der Abdeckeinrichtung 1 und der Kassette 60 besteht in manchen erfindungsgemäßen Ausführungsformen keine stoffschlüssige Verbindung.

Die Kodierstruktur 3 trägt zu einer stabilen Verbindung der Berührschutzvorrichtung 10 an der Kassette 60 bei.

Die Kodierstruktur 3 hat eine Form, die mit der Form oder Kontur eines Randes oder eines Abschnitts der Kassette 60 korreliert oder sich mit diesem ergänzt oder komplementär zu diesem ist. Die Kodierstruktur 3 ist im gezeigten Ausführungsbeispiel wellenförmig. Dies bewirkt einerseits, dass die Verbindung zwischen Berührschutzvorrichtung 10 und der im Randbereich ebenfalls wellenförmig verlaufenden Kassette 60 stabil ist, d. h. es verhindert beispielsweise das Verrutschen der Berührschutzvorrichtung 10 oder ihr Verdrehen um die Achse ihre Verbindung mit der Kassette 60, hier um den Verbindungsabschnitt 2. Andererseits verhindert die Kodierstruktur 3, dass die Berührschutzvorrichtung 10 während des Herstellungsprozesses in einer anderen Position als beabsichtigt an der Kassette 60 angebracht wird. Somit kann ein für den Berührschutz unwirksames Anbringen der Berührschutzvorrichtung 10 vorteilhaft verhindert werden.

Die Fixierungseinrichtung 4 der Berührschutzvorrichtung 10 ist optional vorgesehen. Sie dient, falls vorhanden, zum lösbaren Aufnehmen beispielsweise von Zu- und Ablaufschläuchen. Die Fixierungseinrichtung 4 hält die Schläuche in einer gewünschten Stellung zur Kassette 60. Sie kann so die Beschädigung der Schläuche durch Abknicken oder deren Kontamination durch ungewolltes Lösen der Schläuche von der Kassette 60 verhindern.

In Fig. 1 ist die Fixierungseinrichtung 4 dreiteilig ausgeführt. Zwischen den einzelnen der - beispielsweise drei - Abschnitte können weitere Gegenstände wie Schlauchklemmen vorübergehend mittels Reibhaftung gehalten werden. Dies kann einem Schutz der Gegenstände bis zum Gebrauch der Kassette 60 dienen.

**Fig. 2** zeigt die Berührschutzvorrichtung 10 in einem Schnitt entlang der gestrichelten Linie x - x aus Fig. 1 von der Seite. Die Fixierungseinrichtung 4 ist hier turmartig ausgestaltet.

In der hier dargestellten Ausführungsform der Berührschutzvorrichtung 10 ist die Fixierungseinrichtung 4 auf nur einer Seite der Berührschutzeinrichtung 10 angeordnet. Die Berührschutzvorrichtung 10 weist in der hier dargestellten Ausführungsform auf einer Seite weder Abschnitte des Verbindungsabschnitts 2, der Kodierstruktur 3 noch der Fixiereinrichtung 4 auf. In einigen erfindungsgemäßen Ausführungsformen weist die Berührschutzvorrichtung 10 auf einer Seite (Ober- oder Unterseite) überhaupt keine erhabenen Strukturen auf.

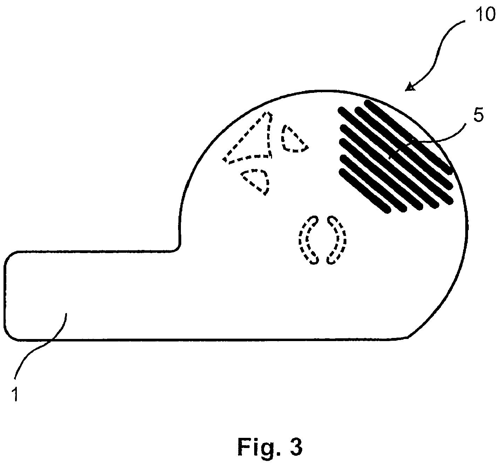

**Fig. 3** zeigt die Berührschutzvorrichtung der Fig. 1 und 2 von oben oder in einer Draufsicht. Auf dieser Seite der Berührschutzeinrichtung 10 ist eine geriffelte Fläche des Griffabschnitts 5 angeordnet. Der Griffabschnitt 5 weist damit eine Struktur oder Oberflächenstruktur auf, welche ein rutschsicheres Halten des Griffabschnitts ermöglicht, beispielsweise als Ring, Noppen, Lasche etc. Der Griffabschnitt 5 kann zu seinem besseren Greifen jede dem Stand der Technik bekannte Ausgestaltung haben. Eine derartige Ausgestaltung oder Oberflächenstruktur ist allerdings wiederum nur optional vorgesehen.

Mittels des Griffabschnitts 5 entfernt oder löst der Benutzer die Berührschutzvorrichtung 10 von der Kassette 60 vor dem Einlegen der Kassette in eine Fluidbehandlungsvorrichtung.

Nur strichliniert sind der Verbindungsabschnitt 2 und die Fixiereinrichtung 4 angedeutet. Diese erstrecken sich nicht auf die in Fig. 3 betrachtete Seite der Berührschutzvorrichtung 10.

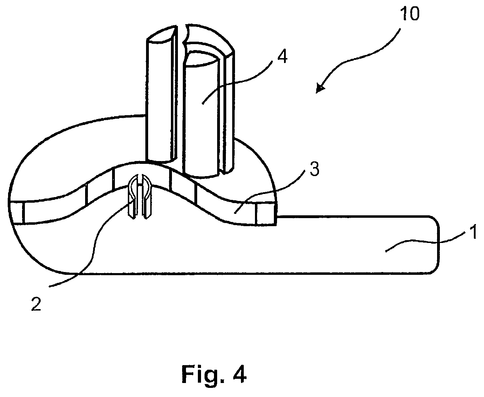

**Fig. 4** zeigt eine perspektivische Darstellung der Berührschutzvorrichtung 10 mit Blick auf die im Gebrauch einer Kassette 60 zugewandte Seite.

**Fig. 5** zeigt in perspektivischer Darstellung die Berührschutzvorrichtung 10 der vorangegangenen Figuren, verbunden mit einer erfindungsgemäßen Kassette 60.

Eine mögliche Ausgestaltung der Berührschutzvorrichtung weist, wie abgebildet, eine plattenförmige Abdeckeinrichtung 1 auf, welche genau eine Konnektionsstelle 41 komplett abdeckt. In anderen erfindungsgemäßen Ausführungsformen ist die Berührschutzvorrichtung beispielsweise T-förmig und deckt zwei oder mehr Konnektionsstellen gleichzeitig ab. Auch andere beliebige Formen als die hier gezeigte sind erfindungsgemäß vorgesehen.

Wenn die Berührschutzvorrichtung 10 mit der Kassette 60 verbunden ist, wie dies in Fig. 5 gezeigt ist, steckt der in Fig. 5 nur zeichnerisch angedeutete Verbindungsabschnitt 2 beispielsweise in einer nicht dargestellten Zentrieröffnung oder Halteöffnung der Kassette 60, vorgesehen zu ihrem Zentrieren oder Halten in einer Fluidbehandlungsvorrichtung (eine nicht mit der Berührschutzvorrichtung 10 verbundene Öffnung 42 dieser Art ist am linken Rand der Kassette 60 gezeigt). Die Kassette 60 kann in diesem Zustand nicht an der Fluidbehandlungsvorrichtung angebracht werden, da die Zentrieröffnung nicht frei zugänglich liegt. Da allerdings ein freier Zugang zur Zentrieröffnung Voraussetzung für das Anbringen der Kassette 60 an der Fluidbehandlungsvorrichtung ist, muss die Berührschutzvorrichtung 10 bewusst entfernt werden.

Die Berührschutzvorrichtung 10 kann auch derart ausgestaltet sein, beispielsweise durch ihre Dimensionen oder ihre Art der Verbindung zur Kassette 60, dass die Kassette 60 mit aufgesetzter Berührschutzvorrichtung 10 zwar an der Fluidbehandlungsvorrichtung angebracht werden kann, jedoch etwa die Tür der Vorrichtung nicht geschlossen werden kann, bevor die Berührschutzvorrichtung 10 von der Kassette 60 gelöst wird. Auch dies dient dazu sicherzustellen, dass die Berührschutzvorrichtung 10 vor Gebrauch der Kassette 60 zuverlässig entfernt wurde.

Das Lösen der Berührschutzvorrichtung 10 erfolgt ohne nennenswerte Kraftanwendung. Es erfolgt ferner unter vermindertem Risiko der unbeabsichtigten Kontamination der Konnektionsstelle 41 der Kassette 60, etwa durch Berühren mit der Hand, da der Griffabschnitt 5 der Berührschutzvorrichtung 10 von der Abdeckeinrichtung 1 beabstandet ist.

Ferner ragt der Griffabschnitt 5 über den Rand der Kassette 60 hinaus, um dem Benutzer das Entfernen der Berührschutzvorrichtung 10 zu erleichtern.

### Bezugszeichenliste

- 10: Berührschutzvorrichtung
- 1: Abdeckeinrichtung
- 2: Verbindungsabschnitt
- 3: Kodierstruktur
- 4: Fixierungseinrichtung
- 5: Griffabschnitt
- 41: Konnektionsstelle
- 42: Zentrieröffnung oder Halteöffnung
- 60: fluidführende Kassette

## Patentansprüche

1. Medizinische fluidführende Blutkassette (60) für eine medizinische Fluidbehandlung mit wenigstens einer Konnektionsstelle (41) und wenigstens einer Berührschutzvorrichtung (10) zum Abdecken der Konnektionsstelle (41) der medizinischen fluidführenden Kassette (60), wobei die Berührschutzvorrichtung (10) wenigstens eine Abdeckeinrichtung (1) zum Abdecken der Konnektionsstelle (41) vor Gebrauch der Kassette (60), und wenigstens einen Verbindungsabschnitt (2) zum lösbaren Verbinden mit oder Halten der Berührschutzvorrichtung (10) an der Kassette (60) aufweist,
wobei die Verbindung mittels Verbindungsabschnitt (2) mittels Einrasten oder Einklemmen erfolgt;
**dadurch gekennzeichnet, dass** der Verbindungsabschnitt (2) der
Berührschutzvorrichtung (10) diese mit einer Halteöffnung oder einer Zentrieröffnung (42) der Kassette (60) für deren Aufnahme an einer Blutbehandlungsvorrichtung verbindet; und
dass zwischen der Abdeckeinrichtung (1) der Berührschutzvorrichtung (10) in ihrem über der Konnektionsstelle (41) angeordneten Zustand und der Konnektionsstelle (41) keine stoffschlüssige Verbindung besteht.

2. Blutkassette (60) nach Anspruch 1, wobei die Berührschutzvorrichtung (10) wenigstens eine Kodierstruktur (3) zum formbedingt kodierten Anordnen der Berührschutzvorrichtung (10) an der fluidführenden Kassette (60) aufweist.

3. Blutkassette (60) nach einem der vorangegangenen Ansprüche, wobei die Berührschutzvorrichtung (10) wenigstens eine Fixierungseinrichtung (4) zum lösbaren Aufnehmen oder Halten von Zu- und/oder Ablaufschläuchen der fluidführenden Kassette (60) aufweist.

4. Blutkassette (60) nach Anspruch 3, wobei die Fixierungseinrichtung (4) auf nur einer Seite der Berührschutzvorrichtung (10) angeordnet ist.

5. Blutkassette (60) nach einem der vorangegangenen Ansprüche, wobei die Berührschutzvorrichtung (10) wenigstens einen Griffabschnitt (5) zum Greifen der Berührschutzvorrichtung (10) aufweist, um diese von der fluidführenden Blutkassette (60) zu lösen.

6. Blutkassette (60) nach Anspruch 5, wobei die Abdeckeinrichtung (1) nicht auf dem Griffabschnitt (5) vorliegt.

7. Blutkassette (60) nach einem der vorangegangenen Ansprüche, wobei die Berührschutzvorrichtung (10) einteilig hergestellt ist.

8. Blutkassette (60) nach einem der vorangegangenen Ansprüche, wobei die Berührschutzvorrichtung (10) klebstofffrei mit der Blutkassette (60) verbunden ist.

9. Blutkassette (60) nach einem der vorangegangenen Ansprüche, wobei die Berührschutzvorrichtung (10) einen Abschnitt aufweist, welcher über einen Rand der Blutkassette (60) hinausragt.

10. Blutkassette (60) nach einem der vorangegangenen Ansprüche, wobei der Griffabschnitt (5) der Berührschutzvorrichtung (10) auf dem über den Rand der Blutkassette (60) hinausragenden Abschnitt angeordnet ist.

11. Blutkassette (60) nach einem der vorangegangenen Ansprüche, wobei der Verbindungsabschnitt (2) der Berührschutzvorrichtung (10) im Gebrauch das Anbringen der Blutkassette (60) an einer Blutbehandlungsvorrichtung oder das Schließen einer Abdeckung mechanisch verhindert.

12. Blutkassette (60) nach einem der vorangegangenen Ansprüche, wobei die Berührschutzvorrichtung (10) zusammen mit der Blutkassette (60) sterilisiert ist.

13. Blutkassette (60) nach einem der vorangegangenen Ansprüche, mit einer Randkontur, welche entsprechend einer Kontur der Kodierstruktur (3) der Berührschutzvorrichtung (10) ausgestaltet ist.

14. Blutkassette (60) nach einem der vorangegangenen Ansprüche, wobei wenigstens eine Konnektionsstelle (41) eine Verbindungsstelle für eine Substituatleitung ist.

## Claims

1. A medical fluid-conducting blood cassette (60) for a medical fluid treatment with at least a connection point (41) and at least a contact protection apparatus (10) for covering the connection point (41) of the medical fluid-conducting cassette (60), wherein the contact protection apparatus (10) comprises at least one covering device (1) for covering the connection point (41) before the use of the cassette (60), and at least one connection section (2) for detachably connecting or holding the contact protection apparatus (10) on the cassette (60),
wherein the connection is effected via the connection section (2) by means of latching or clamping,
**characterized in that** the connection section (2) of the contact protection apparatus (10) connects the contact protection apparatus (10) with a retaining hole or a centering hole (42) of the cassette (60) for its intake at a blood treatment apparatus; and
**in that** there is no firmly bonded connection between the covering device (1) of the contact protection apparatus (10) and the connection point (41) when the covering device (1) is arranged over or across the connection point (41).

2. The blood cassette (60) according to claim 1, wherein the contact protection apparatus (10) comprises at least one encoding structure (3) for an arrangement which is encoded on the fluid-conducting cassette (60) in relation to the shape of the contact protection apparatus (10).

3. The blood cassette (60) according to one of the preceding claims, wherein the contact protection apparatus (10) comprises at least one fixing device (4) for detachable intaking or retaining inlet and/or outlet tubes of the fluid-conducting cassette (60).

4. The blood cassette (60) according to claim 3, wherein the fixing device (4) is arranged only on one side of the contact protection apparatus (10).

5. The blood cassette (60) according to one of the preceding claims, wherein the contact protection apparatus (10) comprises at least one grip area (5) for grasping the protection apparatus (10) in order to detach it from the fluid-conducting blood cassette (60).

6. The blood cassette (60) according to claim 5, wherein the covering device (1) is not present on the grip area (5).

7. The blood cassette (60) according to one of the preceding claims, wherein the contact protection apparatus (10) is produced to be one-piece.

8. The blood cassette (60) according to one of the preceding claims, wherein the contact protection apparatus (10) is connected with the blood cassette (60) without adhesives.

9. The blood cassette (60) according to one of the preceding claims, wherein the contact protection apparatus (10) comprises a section which protrudes from an edge of the blood cassette (60).

10. The blood cassette (60) according to one of the preceding claims, wherein the grip area (5) of the contact protection apparatus (10) is arranged on the section protruding from the edge of the blood cassette (60).

11. The blood cassette (60) according to one of the preceding claims, wherein the connection section (2) of the contact protection apparatus (10) mechanically prevents the blood cassette from being mounted to a blood treatment apparatus or a cover being sealed during use.

12. The blood cassette (60) according to one of the preceding claims, wherein the contact protection apparatus (10) is or was sterilized together with the blood cassette (60).

13. The blood cassette (60) according to one of the preceding claims, with an edge contour which is designed corresponding to a contour of the encoding structure (3) of the contact protection apparatus (10).

14. The blood cassette (60) according to one of the preceding claims, wherein at least one connection point (41) is a joint for a substituate line.

## Revendications

1. Cassette médicale à sang conductrice de fluides (60) pour un traitement médical des fluides comprenant au moins un point de connexion (41) et au moins un appareil de protection anti-contact (10) pour protéger le point de connexion (41) de la cassette à sang conductrice de fluides (60), où l'appareil de protection anti-contact (10) présente au moins un dispositif de protection (1) pour protéger le point de connexion (41) avant l'usage de la cassette (60) et au moins une section de connexion (2) pour connecter l'appareil de protection anti-contact (10) à la cassette (60) de façon détachable ou pour maintenir l'appareil de protection anti-contact (10) avec la cassette (60),
où la connexion avec la section de connexion s'effectue au moyen d'un encliquetage ou d'un coincement,
**caractérisée en ce que** la section de connexion (2) de l'appareil de protection anti-contact (10) connecte l'appareil de protection avec une ouverture de retenue ou une ouverture de centrage (42) de la cassette (60) pour sa réception dans un appareil de traitement du sang ; et
**en ce que** le dispositif de protection (1) de l'appareil de protection anti-contact (10) lorsqu'il est disposé en dessus du point de connexion (41) et le point de connexion (41) ne forment pas de liaison solide.

2. Cassette à sang (60) selon la revendication 1, où l'appareil de protection anti-contact (10) présente au moins une structure de codage (3) dont la forme géométrique permet un agencement codé de l'appareil de protection anti-contact (10) avec la cassette conductrice de fluides (60).

3. Cassette à sang (60) selon l'une des revendications précédentes, où l'appareil de protection anti-contact (10) présente au moins un dispositif de fixation (4) pour la réception ou le maintien détachable de tuyaux d'entrée et/ou de sortie de la cassette conductrice de fluides (60).

4. Cassette à sang (60) selon la revendication 3, où le dispositif de fixation (4) est disposé sur l'une des faces de l'appareil de protection anti-contact (10) seulement.

5. Cassette à sang (60) selon l'une des revendications précédentes, où l'appareil de protection anti-contact (10) présente au moins une section de préhension (5) pour saisir l'appareil de protection anti-contact (10) afin de le séparer de la cassette à sang conductrice de fluides (60).

6. Cassette à sang (60) selon la revendication 5, où le dispositif de protection (1) n'est pas présent sur la section de préhension (5).

7. Cassette à sang (60) selon l'une des revendications précédentes, où l'appareil de protection anti-contact (10) est formé d'une seule pièce.

8. Cassette à sang (60) selon l'une des revendications précédentes, où l'appareil de protection anti-contact (10) est connecté à la cassette à sang (60) sans adhésif.

9. Cassette à sang (60) selon l'une des revendications précédentes, où l'appareil de protection anti-contact (10) présente une section dépassant d'un rebord de la cassette à sang (60).

10. Cassette à sang (60) selon l'une des revendications précédentes, où la section de préhension (5) de l'appareil de protection anti-contact (10) est disposée sur la section dépassant du rebord de la cassette à sang (60).

11. Cassette à sang (60) selon l'une des revendications précédentes, où la section de connexion (2) de l'appareil de protection anti-contact (10) empêche mécaniquement lors de son usage la mise en place de la cassette à sang (60) sur un appareil de traitement du sang ou la fermeture d'une protection.

12. Cassette à sang (60) selon l'une des revendications précédentes, où l'appareil de protection anti-contact (10) est stérilisé avec la cassette à sang (60).

13. Cassette à sang (60) selon l'une des revendications précédentes avec un contour formé conformément au contour de la structure de codage (3) de l'appareil de protection anti-contact (10).

14. Cassette à sang (60) selon l'une des revendications précédentes, où au moins un point de connexion (41) est un point de raccord pour une ligne de substitut.
